(19) Europäisches Patentamt
European Patent Office
Office européen des brevets

(11) Veröffentlichungsnummer: **0 532 562 B1**

# (12) EUROPÄISCHE PATENTSCHRIFT

(45) Veröffentlichungstag der Patentschrift: **23.11.94**

(51) Int. Cl.5: **C07J 21/00**, C07J 41/00, C07J 31/00, C07J 1/00, C07J 51/00, C07J 43/00, C07J 33/00

(21) Anmeldenummer: **91910189.9**

(22) Anmeldetag: **03.06.91**

(86) Internationale Anmeldenummer:
**PCT/EP91/01018**

(87) Internationale Veröffentlichungsnummer:
**WO 91/18918 (12.12.91 91/28)**

## (54) VERFAHREN ZUR HERSTELLUNG VON 10-g(b)-H-STEROIDEN.

(30) Priorität: **01.06.90 DE 4018167**

(43) Veröffentlichungstag der Anmeldung:
**24.03.93 Patentblatt 93/12**

(45) Bekanntmachung des Hinweises auf die Patenterteilung:
**23.11.94 Patentblatt 94/47**

(84) Benannte Vertragsstaaten:
**AT BE CH DE DK ES FR GB GR IT LI LU NL SE**

(56) Entgegenhaltungen:
**EP-A- 0 037 973**
**EP-A- 0 145 493**
**EP-A- 0 277 676**

(73) Patentinhaber: **SCHERING AKTIENGESELLSCHAFT**

**D-13342 Berlin (DE)**

(72) Erfinder: **OTTOW, Eckhard**
**Moltkestr. 48**
**D-1000 Berlin 45 (DE)**
Erfinder: **NEEF, Günter**
**Markgraf-Albrecht-Str. 4**
**D-1000 Berlin 31 (DE)**
Erfinder: **CLEVE, Arwed**
**Blumenthalstr. 19**
**D-1000 Berlin 42 (DE)**
Erfinder: **WIECHERT, Rudolf**
**Petzower Str. 8A**
**D-1000 Berlin 39 (DE)**

EP 0 532 562 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 10β-H-11β-(subst. Phenyl)-Steroiden der Formel Ia

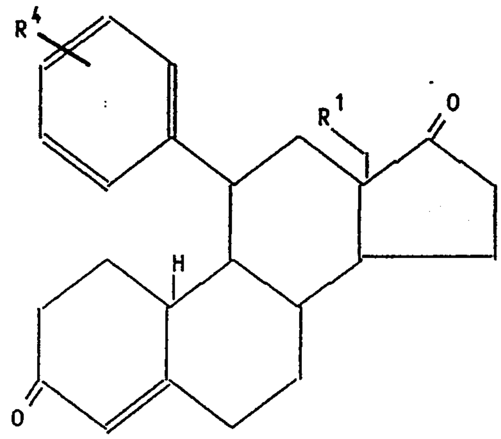

(Ia)

oder der Formel Ib

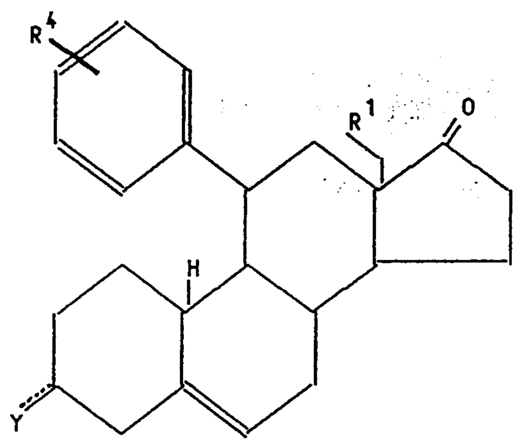

(Ib)

worin
$R^4$ für ein Wasserstoffatom, eine Cyanogruppe, ein Chlor-, Fluor-, Brom-, Jod-atom, für eine Trialkylsilyl-, Trialkylstannylgruppe, für einen geradkettigen oder verzweigten, gesättigten oder ungesättigten $C_1$-$C_8$-Alkyl-, gegebenenfalls geschützten Acyl-oder Alkoxyalkylrest, für eine Aminogruppe

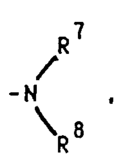

in welcher $R^7$ und $R^8$ unabhängig voneinander ein Wasserstoffatom oder eine $C_1$-$C_4$-Alkylgruppe bedeuten, oder für ein entsprechendes Aminoxid

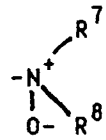 ,

oder für die Gruppierungen $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα

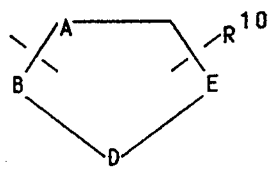 (Iα),

in welchem A ein Stickstoff-, Sauerstoff- oder Schwefelatom, -B-D-E- die Elementenfolge -C-C-C-, -N-C-C- oder -C-N-C- und $R^{10}$ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten $C_1$-$C_8$-Alkyl-, gegebenenfalls geschützten Acyl-oder Alkoxyalkylrest, für eine Aminogruppe

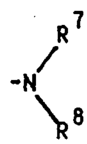 ,

in welcher $R^7$ und $R^8$ unabhängig voneinander ein Wasserstoffatom oder eine $C_1$-$C_4$-Alkylgruppe bedeuten, oder ein entsprechendes Aminoxid

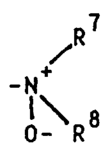 ,

oder die Gruppierung $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten, symbolisieren,
oder für einen Heteroarylrest der Formel Iβ

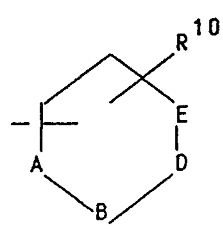 (Iβ),

in welchem A ein Stickstoffatom und -B-D-E- die Elementenfolge -C-C-C-, - N-C-C-, -C-N-C- oder -C-C-N- bedeuten und $R^{10}$ die bereits angegebene Bedeutung hat, oder für einen Phenylrest der Formel Iγ

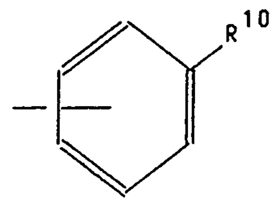

(Iγ),

worin $R^{10}$ die bereits angegebene Bedeutung hat, stehen,

Y eine geschützte Ketogruppe oder eine geschützte Hydroxygruppe und ein Wasserstoffatom sowie

$R^1$ ein Wasserstoffatom oder eine Methylgruppe bedeuten,

In der deutschen Patentanmeldung P 39 21 059.6 sind erstmals 11β-Aryl-4-estrene der allgemeinen Formel I

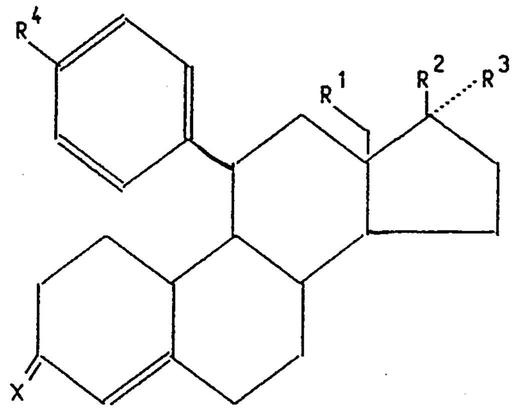

(I)

beschrieben, worin

X für ein Sauerstoffatom, die Hydroxyiminogruppierung >N~OH oder zwei Wasserstoffatome,

$R^1$ für ein Wasserstoffatom oder eine Methylgruppe,

$R^2$ für eine Hydroxygruppe, eine $C_1$-$C_{10}$-Alkoxy- oder $C_1$-$C_{10}$-Acyloxygruppe,

$R^3$ für ein Wasserstoffatom, die Gruppierung

$-(CH_2)_nCH_2Z$, wobei n 0, 1, 2, 3, 4 oder 5 ist, Z ein Wasserstoffatom, die Cyanogruppe oder den Rest $-OR^5$ mit $R = {}^5H$, $C_1$-$C_{10}$-Alkyl oder $C_1$-$C_{10}$-Acyl bedeuten, die Gruppierung $-(CH_2)_m-C{\equiv}C-Y$, wobei m 0, 1 oder 2 und Y ein Wasserstoff-, Fluor-, Chlor-, Brom- oder Jod-Atom, einen $C_1$-$C_{10}$-Hydroxyalkyl-, $C_1$-$C_{10}$-Alkoxyalkyl-, $C_1$-$C_{10}$-Acyloxyalkylrest bedeuten, die Gruppierung $-CH=CH-(CH_2)_kCH_2R^6$, wobei k 0, 1 oder 2 und $R^6$ ein Wasserstoffatom, eine Hydroxygruppe, einen $C_1$-$C_4$-Alkoxy- oder $C_1$-$C_4$-Acyloxyrest bedeuten, oder aber $R^2$ und $R^3$ gemeinsam für einen Rest der Formel

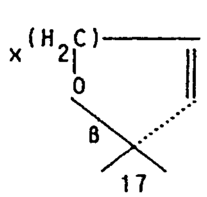 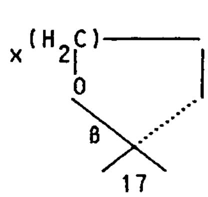 oder 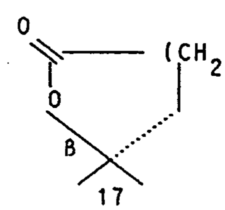 wobei x = 1 oder 2 ist

$R^4$ für ein Wasserstoffatom, für einen geradkettigen oder verzweigten, gesät tigten oder ungesättigten $C_1$-$C_8$-Alkyl-, -Acyl- oder Alkoxyalkylrest, für eine Aminogruppe

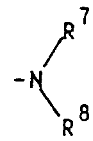 ,

in welcher $R^7$ und $R^8$ unabhängig voneinander ein Wasserstoffatom oder eine $C_1$-$C_4$-Alkylgruppe bedeuten, oder für ein entsprechendes Aminoxid

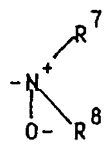 ,

oder für die Gruppierung -$OR^9$, in welcher $R^9$ ein Wasserstoffatom, eine Methyl-, Ethyl-, Propyl-, Methoxyphenyl-, Allyl- oder eine 2-Dimethylaminoethylgruppe bedeuten, oder für einen Heteroarylrest der Formel I$\alpha$

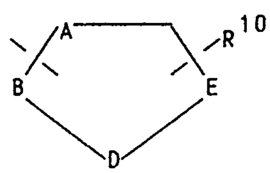 (I$\alpha$),

in welchem A ein Stickstoff-, Sauerstoff- oder Schwefelatom, -B-D-E- die Elementenfolge -C-C-C-, -N-C-C- oder -C-N-C- und $R^{10}$ ein Wasserstoffatom,
eine Cyano-, Trialkylsilyl-, Trialkylstannyl- oder Aminogruppe

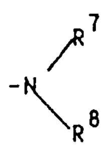

oder den Rest -$OR^9$ bzw. -$SR^9$ mit $R^7$, $R^8$ und $R^9$ in der bereits angegebenen Bedeutung symbolisieren, oder für einen Heteroarylrest der Formel I$\beta$

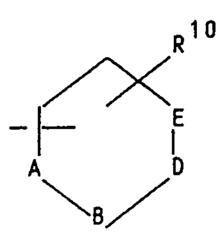 (I$\beta$),

in welchem A ein Stickstoffatom und -B-D-E- die Elementenfolge -C-C-C-, -N-C-C-, -C-N-C- oder -C-C-N- bedeuten und $R^{10}$ die bereits angegebene Bedeutung hat,
oder für einen Phenylrest der Formel I$\gamma$

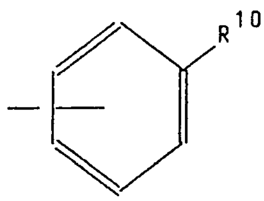

(Iγ),

worin $R^{10}$ die bereits angegebene Bedeutung hat, stehen, sowie deren pharmakologisch verträgliche Additionssalze mit Säuren.

Die neuen Verbindungen besitzen in erster Linie wegen ihrer starken antigestagenen Eigenschaften großes Interesse. Sie werden gemäß der deutschen Patentanmeldung P 39 21 059.6 (bzw. EP-A-0 404 283) hergestellt:

Behandlung von Verbindungen der allgemeinen Formel II

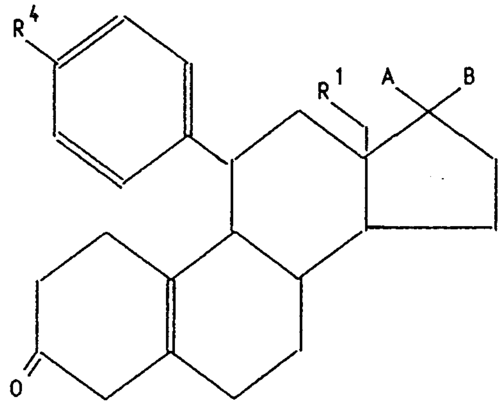

(II),

worin
$R^1$ und $R^4$ die in Formel I angegebene Bedeutung haben,
A für eine β-Hydroxygruppe oder den Rest $R^2$ und
B für ein α-Wasserstoffatom, einen α-ständigen Rest $R^3$ oder
A und B gemeinsam für ein Ketosauerstoffatom stehen,
mit Säure in einem inerten Lösungsmittel unter Erhitzen führt zu Verbindungen der allgemeinen Formel Ia'

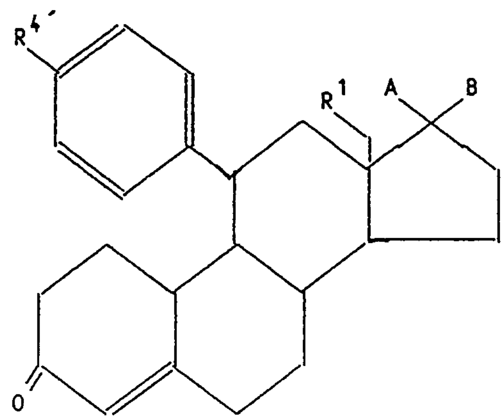

(Ia'),

worin $R^1$, A und B die in Formel II angegebene Bedeutung haben und $R^{4'}$ dieselbe Bedeutung wie $R^4$ in Formel I hat, mit der Maßgabe, daß $R^4$ unter den genannten drastischen Reaktionsbedingungen stabil ist.
Vorzugsweise wird zur Isomerisierung auf eine Temperatur zwischen 80 und 120 °C erhitzt, und zwar in einem inerten Lösungsmittel wie etwa Toluol.
Die Reaktionsdauer beträgt mindestens 45 Minuten, kann aber erforderlichenfalls 24 Stunden oder mehr

betragen.
Als Säuren eignen sich sowohl Mineral- als auch organische Säuren; von letzteren ist p-Toluolsulfonsäure bevorzugt.

Es wurde nun gefunden, daß die Verbindungen der allgemeinen Formeln Ia und Ib, die als Ausgangsprodukte für die Herstellung der 10β-H-Steroide der allgemeinen Formel I geeignet sind, bequem herstellen lassen, indem man eine Verbindung der Formel III

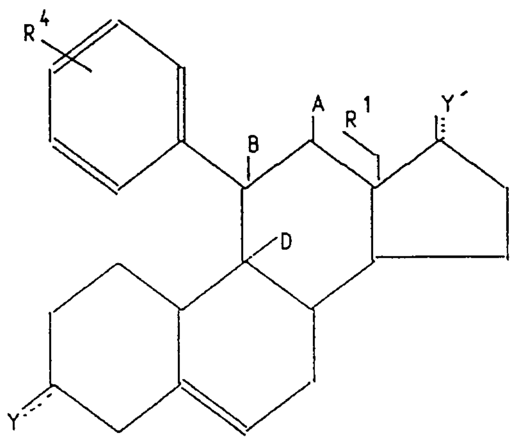

(III)

worin
A und B gemeinsam eine zusätzliche Bindung und
D ein Wasserstoffatom oder
B und D gemeinsam eine zusätzliche Bindung und
A ein Wasserstoffatom
bedeuten und
$R^4$, Y, Y' sowie $R^1$ die in den Formeln Ia bzw. Ib angegebene Bedeutung haben, zu einer Verbindung der allgemeinen Formel IIa

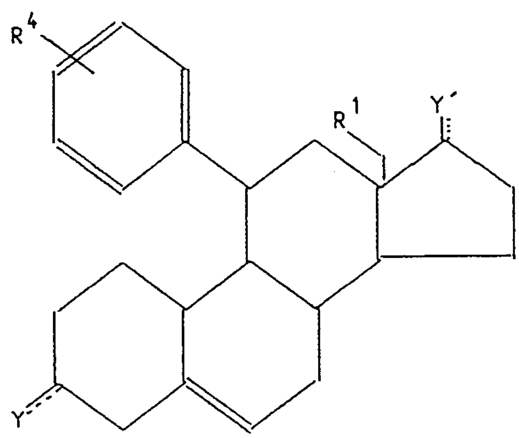

(IIa),

worin $R^4$, Y, Y' und $R^1$ die bereits angegebene Bedeutung haben, mit einem elektropositiven Metall in einem elektronensolvatisierenden Lösungsmittel reduziert und anschließend gegebenenfalls diese Verbindung der allgemeinen Formel IIa entweder mit Salzsäure, Schwefelsäure, Phosphorsäure, Toluolsulfonsäure oder mit einer anderen starken Mineral Säure zu einer Verbindung der allgemeinen Formel Ia oder mit Essigsäure, Oxalsäure oder einer anderen organischen Säure partiell zu einer Verbindung der allgemeinen Formel Ib gespalten wird.

Bei der Reduktion von III bildet sich die 11β-Arylverbindung IIa (stereoselektive Reduktion).
Zur Reduktion der 9(11)- bzw. 11(12)-Doppelbindung in III kommt erfindungsgemäß die Reduktion mit einem elektropositiven Metall in einem eletronensolvatisierenden Lösungsmittel oder in einem einen

Lösungsvermittler enthaltenden Lösungsmittel infrage. Als elektronensolvatisierendes Lösungsmittel kommt in erster Linie Ammoniak inbetracht.

Für die Reduktion genügen bereits equimolare Mengen Reduktionsmittel; es kann jedoch auch ein beträchtlicher Überschuß Reduktionsmittel verwendet werden, ohne daß das aromatische System und/oder die 5,6-Doppelbindung angegriffen werden/wird.
Als elektropositive Metalle sind alle für eine Birch-Reduktion geeigneten Metalle verwendbar. Erfindungsgemäß sind Lithium, Kalium, Natrium und Calcium - und von diesen Lithium insbesondere - bevorzugt.

Die vorliegende Erfindung betrifft auch die Verbindungen der allgemeinen Formeln IIa, Ia und Ib. Sie lassen sich nach der in der P 39 21 059.6 und in der korrespondierenden europäischen Patentanmeldung EP-A-0 404 283 angegebenen Weise zu den wertvollen Endprodukten der allgemeinen Formel I weiterverarbeiten.
Die geschützten Ketogruppen der bei der Birch-Reduktion entstandenen Verbindung der allgemeinen Formel IIa können je nach beabsichtigter Weiterverarbeitung sukzessive abgespalten werden.

Entspricht der 4-Arylsubstituent in der Verbindung der allgemeinen Formel IIa bereits dem Substituenten $R^4$ im Endprodukt der Formel I, so wird zunächst mit einer schwachen Säure (Essigsäure, Oxalsäure) die Ketoschutzgruppe in 17-Position abgespalten, die Substituenten $R^2$ und $R^3$ eingeführt und dann mit einer starken Säure die Schutzgruppe in 3-Position abgetrennt. Als starke Säuren seien beispielsweise Salzsäure, Schwefelsäure, Phosphorsäure oder Toluolsulfonsäure genannt. Danach können noch weitere Reaktionen zur Funktionalisierung folgen, beispielsweise die Hydrierung einer C-C-Dreifach- zu einer C-C-Doppelbindung.

Wenn der 4-Arylsubstituent für eine Methoxygruppe steht und $R^4$ im Endprodukt der allgemeinen Formel I eine andere Bedeutung haben soll, geht man so vor, daß am 11$\beta$-Phenylrest zuerst der letztendlich gewünschte Substituent $R^4$ wie in der P 39 21 059.6 beschrieben aufgebaut, anschließend die 17-Ketogruppe freigesetzt, $R^2$ und $R^3$ eingeführt und dann die 3-Ketoschutzgruppe abgespalten wird. Diese Vorgehensweise ist dann angebracht, wenn der Substituent $R^4$ nicht ohne weiteres bereits bei der Herstellung des Ausgangsproduktes der allgemeinen Formel III am Aromaten quasi "miteingeführt" werden kann.

Behandlung einer Verbindung der allgemeinen Formel IIa mit einer starken Säure führt direkt zum entsprechenden 3,17-Diketon der allgemeinen Formel Ia.

Aus einer Verbindung der allgemeinen Formel Ia kann durch Thioketalisierung nach gängigen Methoden eine Verbindung der allgemeinen Formel III hergestellt werden, die für das in der P 39 21 059.6 beschriebene Verfahren benötigt wird.

Es ist auch möglich, aus der 4-Methoxy-substitierten Arylverbindung der allgemeinen Formel IIa, gegebenenfalls nach selektiver Entfernung der 17-Ketoschutzgruppe, die 4-Hydroxyarylverbindungen herzustellen, diese in die entsprechende Perfluoralkylsulfonyloxyverbindung zu überführen, in diese in der bereits vorstehend beschriebenen Weise entweder direkt durch übergangsmetallisierte Kupplung mit einer entsprechenden, den Rest $R^4$ enthaltenden Verbindung der allgemeinen Formel IV

$R^4$-K (IV),

worin $R^4$ die letztlich für diesen Substituenten in der Formel Ia, Ib oder IIa gewünschte Bedeutung hat und K für einen der Reste

$-B(Alkyl)_2$
$-Sn(Alkyl)_3$ } Alkyl = $C_1$-$C_4$-Alkylrest
$-B(OH)_2$
$-ZnHal$
$-MgHal$ } Hal = Cl, Br, J

steht oder indirekt über die mit Hexabutyldizinn hergestellte 4-(Trialkylzinn)-arylverbindung den letztendlich gewünschten Rest $R^4$ einzubringen und dann noch vorhandene Schutzgruppen abzuspalten.
Durch nachfolgende Thioketalisierung gelangt man wiederum zu für das in P 39 21 059.6 beschriebene Verfahren geeigneten Ausgangsverbindungen.

Die Herstellung der erfindungsgemäß zu verwendenden Ausgangsprodukte der allgemeinen Formel III (für $R^4$ = $OCH_3$) geht aus den nachfolgenden Beispielen Ia), IIa) und Ib), IIb) hervor.

Durch Variation des Substituenten am Phenylring der zur Kupplung mit dem (11-Trifluormethylsulfonyloxy)-substituierten Steroid verwendeten Phenylboronsäure lassen sich in analoger Weise weitere Verbindungen der allgemeinen Formel III herstellen (Y. Hoshino, N. Miyaura und A. Suzuki, Bull. Chem. Soc. Jpn. 61, 3008 (1988); H. Matsubasa, K. Seto, T. Tahara und S. Takahashi, Bull. Chem. Soc. Jpn. 62, 3896 (1989).

Die nachfolgenden Beispiele dienen der näheren Erläuterung der Erfindung:

**Experimenteller Teil:**

Die Herstellung der benötigten Ausgangsprodukte ist in der gleichzeitig eingereichten internationalen Patentanmeldung WO91/18917 beschrieben.

**3,3;17,17-Bis-(ethylendioxy)-11β-(4-methoxyphenyl)-5-estren**

800 ml Ammoniak werden bei -70 °C kondensiert und mit 1,39 g Lithium versetzt. Nach Auftreten der charakteristischen Blaufärbung werden 18,6 g (40 mmol) 17,17-Bis-(ethylendioxy)-11-(4-methoxyphenyl)-5,9(11)-estradiengelöst in 400 ml Tetrahydrofuran zugetropft. Nach 20-minütigem Nachrühren zersetzt man das überschüssige Lithium durch Zugabe von Wasser, dampft das Ammoniak ab, gießt das Reaktionsgemisch auf gesättigte Ammoniumchloridlösung und extrahiert die wäßrige Phase mit Ethylacetat. Die vereinigten organischen Phasen werden mit gesättigter Natriumchloridlösung gewaschen, über Natriumsulfat getrocknet und am Vakuum eingeengt. Nach Chromatographie des Rohprodukts an Kieselgel mit einem Gemisch aus Etylacetat/Hexan werden 15,2 g 3,3;17,17-Bis-(ethylendioxy)-11β-(4-methoxyphenyl)-5-estren und 1,4 g 17,17-Bis-(ethylendioxy)-11-(4-hydroxyphenyl)-5,9(11)-estradien*als weiße Schäume isoliert.

Exemplarisch ist die Darstellung einiger weiterer Verbindungen analog der obigen Vorschrift in der nachstehenden Tabelle aufgeführt :

* Fp. = 168-170 °C (Ethylacetat); $[\alpha]^{20}_D$ = - 11° ($CHCl_3$;c=0,505)

| Produkt | Ausbeute [%] | Physikalische Daten |
|---|---|---|
| 3,3;17,17-Bis-(ethylendioxy)-11β-(4-methoxyphenyl)-5-estren | 81 | Fp. = 187-188 °C (Diisopropylether)<br>$[\alpha]^{20}_D$ = + 2 ° ($CHCl_3$;c = 0,51) |
| 3,3;17,17-Bis-(ethylendioxy)-11β-(4-methylphenyl)-5-estren | 89 | Fp. = 200-201 °C (Diisopropylether)<br>$[\alpha]^{20}_D$ = + 10 ° ($CHCl_3$;c = 0,375) |
| 3,3;17,17-Bis-(ethylendioxy)-11β-[4-(2-propenyl)phenyl)-5-estren | 85 | $^1$H-NMR ($CDCl_3$) δ [ppm]: 7,25 (2H,d J = 9,5Hz,H-aromatisch); 7,04 (2H,d J = 9,5Hz,H-aromatisch); 5,9-6,07 (1H,m,H-CH = ); 5,53 (1H, d J = 5Hz breit,H-6); 5,0-5,1 (2H,m, H-$CH_2$ = ); 3,42 (1H,t J = 5,5Hz breit,H-11); 3,34 (2H,d J = 6Hz,H-$CH_2$-Ar); 0,56 (3H,s,H-18) |
| 11β-[4-(Dimethylamino)phenyl]-3,3;17,17-bis-(ethylendioxy)-5-estren | 96 | $^1$H-NMR ($CDCl_3$ δ [ppm]: 7,19 (2H,d J = 9Hz,H-aromatisch); 6,64 (2H,d J = 9Hz,H-aromatisch); 5,53 (1H,m,H-6); 3,36 (1H, t J = 5,5Hz breit,H-11); 2,92 (6H,s, H-$NMe_2$); 2,41 (1H,m,H-10); 0,60 (3H,s,H-18) |

**11β-(4-Methoxyphenyl)-4-estren-3,17-dion**

1 g 3,3;17,17-Bis-(ethylendioxy)-11β-(4-methoxyphenyl)-5-estren werden in 50 ml Aceton gelöst und unter Schutzgas mit 2,5 ml 4 n wäßriger Salzsäure versetzt. Nach dreistündigem Rühren bei Raumtemperatur und einstündigem bei 40 °C wird das Reaktionsgemisch auf kalte gesättigte Natriumhydrogencarbonatlösung gegossen und die wäßrige Phase mehrmals mit Methylenchlorid extrahiert. Die vereinigten organischen Phasen werden über Natriumsulfat getrocknet und am Vakuum eingeengt. Der Rückstand wird an Kieselgel mit einem Gemisch aus Ethylacetat/Hexan chromatographiert. Man erhält 683 mg 11β-(4-Methoxyphenyl)-4-estren-3,17-dion als weißen Schaum.
Fp. = 155-156 °C (Diisopropylether); $[\alpha]^{20}_D$ = + 169 ° ($CHCl_3$;c = 0.505)

**3,3-(Ethylendioxy)-11β-(4-methoxyphenyl)-5-estren-17-on**

14 g Kieselgel werden in 30 ml Methylenchlorid suspendiert mit 1,4 ml gesättigter Oxalsäurelösung versetzt und 15 Minuten nachgerührt. Zu dieser Suspension werden 4,67 g (10 mmol) 3,3;17,17-Bis-(ethylendioxy)-11β-(4-methoxyphenyl)-5-estren gegeben und das Reaktionsgemisch bei Raumtemperatur 4 Stunden nachgerührt. Anschließend wird es über eine Fritte abgesaugt, der Frittenrückstand mit Methanol/Methylenchlorid nachgewaschen und das so erhaltene Filtrat mit gesättigter Natriumhydrogencarbonatlösung ausgeschüttelt. Die organische Phase wird über Natriumsulfat getrocknet und am Vakuum eingeengt. Der Rückstand wird an Kieselgel mit einem Gemisch aus Ethylacetat/Hexan chromatographiert. Man erhält 3,28 g 3,3-(Ethylendioxy)-11β-(4-methoxyphenyl)-5-estren-17-on als weißen Schaum.

Entsprechend der nachstehenden Tabelle lassen sich analog der obigen Vorschrift auch weitere Produkte herstellen :

| Produkt | Ausbeute [%] | Physikalische Daten |
|---|---|---|
| 3,3-(Ethylendioxy)-11β-(4-methoxyphenyl)-5--estren-17-on | 78 | Fp. = 223 °C (Ethylacetat)<br>$[\alpha]^{20}_D$ = + 90 ° ($CHCl_3$;c = 0,505) |
| 3,3-(Ethylendioxy)-11β-(4-methylphenyl)-5-estren-17-on | 81 | Fp. = 165-166 °C (Diisopropylether)<br>$[\alpha]^{20}_D$ = + 98 ° ($CHCl_3$;c = 0,515) |
| 3,3-(Ethylendioxy)-11β-[4-(2-propenyl)phenyl]-5-estren-17-on | 78 | Fp. = 163-165 °C (Diisopropylether)<br>$[\alpha]^{20}_D$ = + 90 ° ($CHCl_3$;c = 0,505) |

**11β-[4-(Dimethylamino)phenyl]-3,3-(ethylendioxy)-5-estren-17-on**

15 g Kieselgel werden in 25 ml Dichlormethan suspendiert, mit 4 ml 3 molarer Salzsäure versetzt und 15 Minuten nachgerührt. Zu dieser Suspension wird eine Lösung von 5,0 g 11β-[4-(Dimethylamino)phenyl]-3,3;17,17-bis-(ethylendioxy)-5-estren in 26 ml Dichlormethan gegeben und das Reaktionsgemisch bei Raumtemperatur 3 Stunden nachgerührt. Anschließend wird es über eine Fritte abgesaugt, der Frittenrückstand mit Methanol/Dichlormethan nachgewaschen und das so erhaltene Filtrat mit gesättigter Natriumhydrogencarbonatlösung ausgeschüttelt. Die organische Phase wird über Natriumsulfat getrocknet und am Vakuum eingeengt. Der Rückstand wird an Kieselgel mit einem Gemisch aus Ethylacetat/Hexan chromatographiert. Man erhält 2,21 g der Titelverbindung als weißen Schaum.
Fp. = 238-140 °C (Hexan/Ethylacetat); IR (KBr): 1738 $cm^{-1}$ (C = O)

**3,3-(Ethylendioxy)-11β-(4-methylphenyl)-17-[3-(tetrahydro-2H-pyran-2-yloxy)prop-1-inyl]-5-estren-17β-ol**

In 360 ml absolutem Tetrahydrofuran werden bei 0 °C 11,2 ml 2-[(2-Propinyl)oxy]tetrahydro-2H-pyran vorgelegt und anschließend mit 45 ml einer 1,6 molaren n-Butyllithiumlösung in Hexan langsam ohne

größere Temperaturerhöhung versetzt. Nach 15-minütigem Nachrühren tropft man bei Eisbadkühlung langsam eine Lösung von 3 g 3,3-(Ethylendioxy)-11β-(4-methylphenyl)-5-estren-17-on, gelöst in 72 ml absolutem Tetrahydrofuran, zu dieser Reaktionsmischung und läßt sie 60 Minuten nachrühren. Danach wird das Reaktionsgemisch auf Wasser gegossen, die wäßrige Phase mit Ethylacetat extrahiert und die organische Phase mit Natriumchloridlösung gewaschen. Nach Trocknen über Natriumsulfat und Einengen der organischen Phase am Vakuum wird der Rückstand an Aluminiumoxid (neutral, Stufe III) chromatographiert. Es werden 3,68 g des 3,3-(Ethylendioxy)-11β-(4-methylphenyl)-17-[3-(tetrahydro-2H-pyran-2-yloxy)-prop-1-inyl]-5-estren-17β-ol als weißer Schaum erhalten.
IR (KBr): 2240 $cm^{-1}$

17β-Hydroxy-11β-(4-methylphenyl)-17-(3-hydroxyprop-1-inyl)-4-estren-3-on

3,42 g 3,3-(Ethylendioxy)11β-(4-methylphenyl)-17-[3-(tetrahydro-2H-pyran-2-yl-oxy)prop-1-inyl]-5-estren-17β-ol werden in 150 ml Aceton gelöst und unter Schutzgas mit 7,5 ml 4 molarer wäßriger Salzsäure versetzt. Nach dreistündigem Rühren bei Raumtemperatur und einstündigem bei 40 °C wird das Reaktionsgemisch auf kalte gesättigte Natriumhydrogencarbonatlösung gegossen und die wäßrige Phase mehrmals mit Methylenchlorid extrahiert. Die vereinigten organischen Phasen werden über Natriumsulfat getrocknet und am Vakuum eingeengt. Der Rückstand wird an Kieselgel mit einem Gemisch aus Ethylacetat/Hexan chromatographiert. Man erhält 2,1 g 17β-Hydroxy-11β-(4-methylphenyl)-17-(3-hydroxyprop-1-inyl)-4-estren-3-on als gelblichen Schaum.
$[\alpha]_D^{22}$ = - 26,6 ° ($CHCl_3$; c = 0,500)
$^1$H-NMR($CDCl_3$) δ: 7,29 ppm (2H,d J = 8,0Hz,H-aromatisch); 7,09 ppm (2H,d J = 8,0Hz,H-aromatisch); 5,86 ppm (1H,s breit,H-4); 4,37 ppm (2H,m,H-$CH_2O$); 3,39 ppm (1H,dd breit J = 6,5Hz und J = 5,0Hz,H-11); 2,85 ppm (1H,m,H-10); 2,32 ppm (3H,s,H-$CH_3$); 0,76 ppm (3H,s,H-18).

17β-Hydroxy-11β-(4-methylphenyl)-17-(3-hydroxyprop-1-(Z)-enyl)-4-estren-3-on

2 g 17β-Hydroxy-11β-(4-methylphenyl)-17-(3-hydroxyprop-1-inyl)-4-estren-3-on werden in 50 ml Tetrahydrofuran gelöst, mit 2 ml Pyridin versetzt und unter Verwendung von 200 mg Palladium (10%-ig) auf Bariumsulfat als Katalysator bei Normaldruck hydriert. Nach Aufnahme eines Äquivalents Wasserstoff wird das Reaktionsgemisch über Celite filtiert, der Filterückstand mit Ethylacetat nachgewaschen und das Filtrat am Vakuum eingeengt. Der Rückstand wird an Kieselgel mit einem Gemisch aus Hexan/Ethylacetat chromatographiert, und man erhält 1,72 g 17β-Hydroxy-11β-(4-methylphenyl)-17-(3-hydroxyprop1-(Z)-enyl)-4-estren-3-on als weißen Schaum.
$[\alpha]_D^{22}$ = + 75,8 ° ($CHCl_3$; c = 0,510)
$^1$H-NMR($CDCl_3$) δ: 7,29 ppm (2H,d J = 8,0Hz,H-aromatisch); 7,09 ppm (2H,d J = 8,0Hz,H-aromatisch); 5,85 ppm (1H,s breit,H-4); 5,71 ppm (1H,ddd J = 12,0Hz und J = 5,5Hz und J = 5,5Hz,H-CH =); 5,62 ppm (1H,d breit J = 12,0Hz,H-CH =); 4,24 ppm (2H,m,H-$CH_2O$); 3,33 ppm (1H,dd breit J = 6,0Hz und J = 5,0Hz,H-11); 2,84 ppm (1H,m,H-10); 2,32 ppm (3H,s,H-$CH_3$); 0,71 ppm (3H,s,H-18).

3,3;17,17-Bis-(ethylendioxy)-11β-(4-hydroxyphenyl)-5-estren

9,33 g 3,3;17,17-Bis-(ethylendioxy)-11β-(4-methoxyphenyl)-5-estren werden in 100 ml absolutem Dimethylformamid gelöst, mit 5,6 g Natriummethanthiolat versetzt und das Reaktionsgemisch 3 Stunden zum Rückfluß erhitzt. Nach Abkühlen wird es auf Wasser gegossen und die wäßrige Phase mit Ethylacetat extrahiert. Die vereinigten organischen Phasen wäscht man mehrmals mit gesättigter Natriumchloridlösung, trocknet sie über Natriumsulfat und engt am Vakuum ein. Der Rückstand wird an Kieselgel mit einem Gemisch aus Ethylacetat/Hexan chromatographiert und man erhält 8,67 g 3,3;17,17-Bis-(ethylendioxy)-11β-(4-hydroxyphenyl)-5-estren als weißen Schaum.
Fp. = 224-225 °C (Diisopropylether); $[\alpha]^{20}_D$ = + 1.5 ° ($CHCl_3$;c = 0.505)

3,3-(Ethylendioxy)-11β-(4-hydroxyphenyl)-5-estren-17-on

a) Analog der obigen Vorschrift zur Methyletherspaltung werden aus 3 g 3,3-(Ethylendioxy)-11β-(4-methoxyphenyl)-5-estren-17-on 1,83 g 3,3-(Ethylendioxy)-11β-(4-hydroxyphenyl)-5-estren-17-on als weißer Schaum erhalten.
b) Analog der obigen Vorschrift zur selektiven Ketalspaltung werden aus 0,6 g 3,3;17,17-Bis-(ethylendioxy)-11β-(4-hydroxyphenyl)-5-estren 453 mg 3,3-(Ethylendioxy)-11β-(4-hydroxyphenyl)-5-estren-17-on

als weißer Schaum erhalten.
Fp. = 315-317 °C unter Zers. (Methylenchlorid); $[\alpha]^{20}_{D}$ = + 93 ° ($CHCl_3$;c=0.515)

3,3-(Ethylendioxy)-11β-(4-trifluormethylsulfonyloxyphenyl)-5-estren-17-on

a) 1,74 g 3,3-(Ethylendioxy)-11β-(4-hydroxyphenyl)-5-estren-17-on werden unter Schutzgas zusammen mit 2,6 g 4-Dimethylaminopyridin in 42 ml absolutem Methylenchlorid gelöst, auf -78 °C gekühlt und mit 0,94 ml Trifluormethansulfonsäureanhydrid gelöst in 6 ml absolutem Methylenchlorid versetzt. Nach einstündigem Nachrühren wird das Reaktionsgemisch auf gesättigte Natriumhydrogencarbonatlösung gegossen und die wäßrige Phase mehrmals mit Methylenchlorid extrahiert. Die vereinigten organischen Phasen wäscht man mit gesättigter Natriumchloridlösung, trocknet sie über Natriumsulfat und engt am Vakuum ein. Der Rückstand wird an Kieselgel mit einem Gemisch aus Ethylacetat/Hexan chromatographiert und man erhält 1,81 g 3,3-(Ethylendioxy)-11β-(4-trifluormethylsulfonyloxyphenyl)-5-estren-17-on.
Fp. = 208-210 °C (Diisopropylether); $[\alpha]^{20}_{D}$ = + 73 ° ($CHCl_3$;c=0.51)
b) Analog der Vorschrift zur Triflatbildung werden aus 3 g 3,3;17,17-Bis-(ethylendioxy)-11β-(4-hydroxyphenyl)-5-estren 3,1 g 3,3;17,17-Bis-(ethylendioxy)-11β-(4-trifluormethylsulfonyloxyphenyl)-5-estren als weißer Schaum erhalten.
Fp. = 91-93 °C (Methanol); $[\alpha]^{20}_{D}$ = + 2 ° ($CHCl_3$;c=0.51)
Analog der obigen Vorschrift zur selektiven Ketalspaltung werden aus 3 g 3,3;17,17-Bis-(ethylendioxy)-11β-(4-hydroxyphenyl)-5-estren 2,1 g 3,3-(Ethylendioxy)-11β-(4-tri-fluormethylsulfonyloxyphenyl)-5-estren-17-on als weißer Schaum erhalten.

3,3-(Ethylendioxy)-11β-[[[4-(nonafluorbutyl)sulfonyl]oxy]phenyl]-5-estren-17-on

1 g 3,3;17,17-Bis-(ethylendioxy)-11β-(4-hydroxyphenyl)-5-estren wird mit 1,9 g 4-(Dimethylamino)pyridin und 2,4 ml Nonafluorbutansulfonylfluorid in 40 ml Dichlormethan unter Schutzgas zwei Tage bei Raumtemperatur gerührt. Dann wird das Reaktionsgemisch auf gesättigte Natriumhydrogencarbonatlösung gegossen und die wäßrige Phase mehrmals mit Methylenchlorid extrahiert. Die vereinigten organischen Phasen wäscht man mit Wasser, trocknet sie über Natriumsulfat und engt am Vakuum ein. Der Rückstand wird an Kieselgel mit einem Gemisch aus Ethylacetat/Hexan chromatographiert und man erhält 1,26 g der Titelverbindung.
Fp. = 131 °C (Methanol); $[\alpha]^{20}_{D}$ = + 2 ° ($CHCl_3$;c=0.52)

3,3-(Ethylendioxy)-11β-(4-vinylphenyl)-5-estren-17-on

1,7 g 3,3-(Ethylendioxy)-11β-(4-trifluormethylsulfonyloxyphenyl)-5-estren-17-on werden in 25 ml absolutem Dimethylformamid gelöst und mit 270 mg Lithiumchlorid und 190 mg Tetrakistriphenylphosphinpalladium versetzt. Nach fünfminütigem Nachrühren wird das Reaktionsgemisch mit 1,15 ml Tributylvinylzinn versetzt, 1 Stunde bei 110 °C unter Schutzgas gerührt, auf Raumtemperatur abgekühlt und mit Ethylacetat verdünnt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Chromatographie des Rückstandes an Aluminiumoxid (neutral, Stufe III) mit einem Gemisch aus Ethylacetat/Hexan ergeben 1,2 g 3,3-(Ethylendioxy)-11β-(4-vinylphenyl)-5-estren-17-on als weißen Schaum.

3,3-(Ethylendioxy)-11β-(4-tributylstannylphenyl)-5-estren-17-on

17,89 g 3,3-(Ethylendioxy)-11β-(4-trifluormethylsulfonyloxyphenyl)-5-estren-17-on werden in 1,27 l absolutem Dioxan gelöst und mit 5,4 g Lithiumchlorid und 2,95 g Tetrakistriphenylphosphinpalladium versetzt. Nach fünfminütigem Nachrühren wird das Reaktionsgemisch mit 33,1 ml Hexabutyldizinn versetzt, 2,5 Stunden bei Rückfluß unter Schutzgas gerührt, auf Raumtemperatur abgekühlt und mit Ethylacetat verdünnt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Chromatographie des Rückstandes an Kieselgel mit einem Gemisch aus Ethylacetat/Hexan ergeben 14,4 g 3,3-(Ethylendioxy)-11β-(4-tributylstannylphenyl)-5-estren-17-on als weißen Schaum.

3,3-(Ethylendioxy)-11β-[4-(3-pyridyl)phenyl]-5-estren-17-on

4,92 g (9,1 mmol) 3,3-(Ethylendioxy)-11β-(4-trifluormethylsulfonyloxyphenyl)-5-estren-17-on werden in einem Gemisch aus 80 ml Toluol und 35 ml Ethanol gelöst und nacheinander mit 0,53 g Palladiumtetrakistriphenylphosphin, 0,77 g Lithiumchlorid, 11 ml 2 molarer Natriumcarbonatlösung und 1,47 g (10 mmol) Diethyl(3-pyridyl)boran versetzt. Das Reaksionsgemisch wird dann eine Stunde bei 110 °C gerührt, auf Raumtemperatur abgekühlt und mit gesättigter Natriumchloridlösung versetzt. Die organische Phase wird abgetrennt, nacheinander mit 5 %-iger Natronlauge und Wasser gewaschen, über Natriumsulfat gewaschen und am Vakuum eingeengt. Der Rückstand wird an Kieselgel mit einem Gemisch aus Ethylacetat/Hexan chromatographiert. Man erhält 3,55 g 3,3-(Ethylendioxy)-11-[4-(3-pyridyl)phenyl]-5-estren-17-on und 382 mg 3,3-(Ethylendioxy)-11-(4-ethylphenyl)-5-estren-17-on als als weiße Schäume.

3,3-(Ethylendioxy)-11β-[4-(2-thienyl)phenyl]-5-estren-17-on

Zu einer Lösung von 423 µl Thiophen in 8 ml Tetrahydrofuran werden langsam bei Raumtemperatur unter Schutzgas 3,3 ml einer 1,6 molaren Lösung von Butyllithium in Hexan getropft. Nach 30 minütigem Nachrühren werden 872 mg trockenen Zink(II)chlorids, gelöst in 5 ml Diethylether, zum Reaktionsgemisch gegeben und eine Stunde bei Raumtemperatur gerührt. Die so erzeugte 2-Thienylzinkchloridlösung wird zu einer Lösung von 480 mg 3,3-(Ethylendioxy)-11β-(4-trifluormethylsulfonyloxyphenyl)-5-estren-17-on und 51 mg Tetrakistriphenylphosphinpalladium in 10 ml Tetrahydrofuran getropft. Das Reaktionsgemisch wird acht Stunden unter Rückfluß gerührt, auf Raumtemperatur abgekühlt und mit gesättigter Ammoniumchloridlösung versetzt. Die wäßrige Phase wird dreimal mit Ethylacetat extrahiert. Die organischen Phasen werden mit gesättigter Natriumchloridlösung gewaschen, über Natriumsulfat getrocknet und am Vakuum eingeengt. Chromatographie des Rückstandes an Kieselgel mit einem Gemisch aus Ethylacetat/Hexan ergibt 380 mg der Titelverbindung als weißen Schaum.

3,3-(Ethylendioxy)-11β-[4'-(methylthio)[1,1'-biphenyl]-4-yl]-5-estren-17-on

1,73 g 3,3-(Ethylendioxy)-11β-(4-trifluormethylsulfonyloxyphenyl)-5-estren-17-on werden in einem Gemisch aus 17 ml Toluol und 5 ml Ethanol gelöst und nacheinander mit 37 mg Palladiumtetrakistriphenylphosphin, 0,28 mg Lithiumchlorid, 4 ml 2m Natriumcarbonatlösung und 0,7 g [4-(Methylthio)phenyl]-boronsäure versetzt. Das Reaktionsgemisch wird dann 6 Stunden bei 95 °C gerührt, auf Raumtemperatur abgekühlt und mit gesättigter Natriumchloridlösung versetzt. Die organische Phase wird abgetrennt, nacheinander mit 5 %-iger Natronlauge und Wasser gewaschen, über Natriumsulfat getrocknet und am Vakuum eingeengt. Der Rückstand wird an Kieselgel mit einem Gemisch aus Ethylacetat/Hexan chromatographiert. Man erhält 1,65 g der Titelverbindung als weißen Schaum.

Entsprechend der nachstehenden Tabelle lassen sich analog den fünf vorgenannten Vorschriften auch weitere Produkte herstellen :

| Aromat | Produkt | Ausbeute [%] | Physikalische Daten |
|---|---|---|---|
| Tri-n-butylvinylzinn | 3,3-Ethylendioxy-11ß-(4-vinylphenyl)-5-estren-17-on | 91 | Fp. = > 330 °C Zers. (Diisopropylether) $[\alpha]_D^{20}$ = +96 ° ($CHCl_3$; c=0,5) |
| Tri-n-butyl-3-furylzinn | 3,3-Ethylendioxy-11ß-[4-(3-furyl)-phenyl]-5-estren-17-on | 82 | Fp. = 204 °C (Diisopropylether) $[\alpha]_D^{20}$ = +83 ° ($CHCl_3$; c=0,515) |
| Diethyl-3-pyridylboran | 3,3-Ethylendioxy-11ß-[4-(3-pyridyl)-phenyl]-5-estren-17-on | 83 | Fp. = 133 °C (Ethylacetat) $[\alpha]_D$ = +73 ° ($CHCl_3$; c=0,5) |
| | 3,3-Ethylendioxy-11ß-(4-ethylphenyl)-5-estren-17-on | 10 | Fp. = 173 °C (Diisopropylether) $[\alpha]_D$ = +94 ° ($CHCl_3$; c=0,5) |
| Diethyl-5-pyrimidinylboran | 3,3-Ethylendioxy-11ß-[4-(5-pyrimidinyl)-phenyl]-5-estren-17-on | 73 | Fp. = 212 °C (Ethylacetat/Hexan) $[\alpha]_D$ = +75 ° ($CHCl_3$; c=0,5) |
| | 3,3-Ethylendioxy-11ß-(4-ethylphenyl)-5-estren-17-on | 8 | |
| Hexabutyldizinn | 3,3-Ethylendioxy-11ß-(4-tri-n-butylstannylphenyl)-5-estren-17-on | 64 | $^1$H-NMR ($CDCl_3$) δ [ppm]: 7,25-7,35 (4H,m,H-aromatisch); 5,57 (1H, m,H-6); 3,44 (1H,tr J=5,5Hz breit,H-11); 0,85 (3H,tr J=7,5Hz, H-$CH_3$); 0,55 (3H,s, H-18) |

| Aromat | Produkt | Ausbeute [%] | Physikalische Daten |
|---|---|---|---|
| 2-Thienylzinkchlorid | 3,3-Ethylendioxy-11ß-[4-(2-thienyl)phenyl]-5-estren-17-on | 90 | Fp. = 214 °C (Ethylacetat/Hexan) $[\alpha]_D^{20}$ = +76 ° ($CHCl_3$; c=0.5) |
| 2-Benzofuranylzinkchlorid | 11β-[4-(2-Benzofuranyl)phenyl]-3,3-ethylendioxy-5-estren-17-on | 91 | $^1$H-NMR ($CDCl_3$) δ [ppm]: 7,77 (2H,d J=9Hz,H-aromatisch); 7,59-7,20 (4H,m,H-Benzofuranyl); 7,45 (2H,d J=9Hz,H-aromatisch); 7,00 (1H,s,H-Furanyl); 5,59 (1H, m,H-6); 3,52 (1H,t breit ,J= 5,5Hz,H-11); 0,63 (1H,s, H-18) |
| (5-Ethyl-2-thienyl)-tributylstannan | 3,3-Ethylendioxy-11ß-[4-(5-ethyl-2-thienyl)phenyl]-5-estren-17-on | 70 | Fp. = 192 °C (Ethylacetat/ Diisopropylether) $[\alpha]_D$ = +71 ° ($CHCl_3$; c=0,5) |
| [4-(Methylthio)phenyl]boronsäure | 3,3-Ethylendioxy-11ß-[4'-(methylthio)[1,1'-biphenyl]-4-yl]-5-estren-17-on | 69 | Fp. = 226 °C (Hexan/Ethylacetat) IR (KBr): 1740 $cm^{-1}$ (C=O) |
| [4-(Dimethylamino)phenyl]boronsäure | 11β-[4'-(Dimethylamino)[1,1'-biphenyl]-4-yl]-3,3-ethylendioxy-5-estren-17-on | 58 | $^1$H-NMR ($CDCl_3$) δ [ppm]: 7,51 (2H,d J=9Hz,H-aromatisch); 7,46 (2H,d J=9Hz,H-aromatisch); 7,37 (2H,d J=9Hz,H-aromatisch); 6,80 (2H,d J=9Hz,H-aromatisch); 5,59 (1H,m,H-6); 3,50 (1H, t breit J=5,5Hz,H-11); 3,00 (6H,s,H-$NMe_2$); 0,63 (3H,s, H-18) |
| [4-(2-Methyl-1,3-dioxolan-2-yl)phenyl]boronsäure | 3,3-Ethylendioxy-11ß-[4'-(2-methyl-1,3-dioxolan-2-yl)[1,1'-biphenyl]-4-yl]-5-estren-17-on | 82 | $^1$H-NMR ($CDCl_3$) δ [ppm]: 7,59 (2H,d J=9Hz,H-aromatisch); 7,55 (2H,d J=9Hz,H-aromatisch); 7,50 (2H,d J=9Hz,H-aromatisch); 7,42 (2H,d J=9Hz,H-aromatisch); 5,60 (1H,m,H-6); 3,52 (1H, t breit J=5,5Hz,H-11); 1,70 (3H,s,H-Methyl); 0,632(3H, s,H-18) |

3,3-(Ethylendioxy)-17-cyanmethyl-11β-[4-(3-pyridyl)phenyl]-5-estren-17β-ol

Zu einer Lösung von 3,31 ml Diisopropylamin in 100 ml absolutem Tetrahydrofuran werden bei - 78 °C unter Schutzgas 13,7 ml einer 1,6 n n-Butyllithiumlösung in Hexan getropft. Nach 30 Minuten werden bei

der gleichen Temperatur 1,13 ml Acetonitril zugegeben. 15 Minuten später wird bei der gleichen Temperatur eine Lösung von 1 g 3,3-(Ethylendioxy)-11β-[4-(3-pyridyl)phenyl]-5-estren-17-on in 90 ml absolutem Tetrahydrofuran zugetropft und weitere zwei Stunden nachgerührt. Dann wird mit gesättigter Ammoniumchloridlösung versetzt und mit Ethylacetat extrahiert. Die vereinigten organischen Phasen werden mit gesättigter Natriumchloridlösung gewaschen, über Natriumsulfat getrocknet und am Vakuum eingeengt. Chromatographie des Rückstandes an Kieselgel mit einem Gemisch aus Ethylacetat/Hexan ergibt 0,95 g 3,3-(Ethylendioxy)-17-cyanmethyl-11β-[4-(3-pyridyl)phenyl]-5-estren-17β-ol als weißen Schaum.
IR (KBr): 2250 $cm^{-1}$

**17β-Hydroxy-17-cyanmethyl-11β-[4-(3-pyridyl)phenyl]-4-estren-3-on**

Analog der obigen Vorschrift zur Ketalspaltung werden aus 0,9 g 3,3-(Ethylendioxy)-17-cyanmethyl-11β-[4-(3-pyridyl)phenyl]-5-estren-17β-ol 0,63 g 17β-Hydroxy-17-cyanmethyl-11β-[4-(3-pyridyl)phenyl]-4-estren-3-on als weißer Schaum erhalten.
Fp. = 173-174 °C; $[\alpha]^{20}_D$ = + 132 ° ($CHCl_3$;c=0.5)

**3,3-(Ethylendioxy)-11β-[4'-(methylsulfinyl)[1,1'-biphenyl]-4-yl]-5-estren-17-on**

494 mg 3,3-(Ethylendioxy)-11β-[4'-(methylthio)[1,1'-biphenyl]-4 yl]-5-estren-17-on werden in einem Gemisch aus 6 ml Tetrahydrofuran, 6 ml Methanol und 2 ml Wasser gelöst und mit 924 mg Natriumperiodat versetzt. Über Nacht wird das Reaktionsgemisch bei Raumtemperatur nachgerührt, dann über Celite filtriert und das Filtrat mit Ethylacetat verdünnt. Die organische Phase wird mit gesättigter Natriumhydrogencarbonatlösung gewaschen, über Natriumsulfat getrocknet und am Vakuum eingeengt. Nach Chromatographie des Rückstands an Kieselgel mit einem Gemisch aus Hexan/Ethylacetat erhält man 346 mg der Titelverbindung als weißen Schaum.
IR (KBr): 1736 $cm^{-1}$ (C=O)

**Patentansprüche**

1. Verfahren zur Herstellung von 10β-H-11β-(subst. Phenyl)-Steroiden der Formel Ia

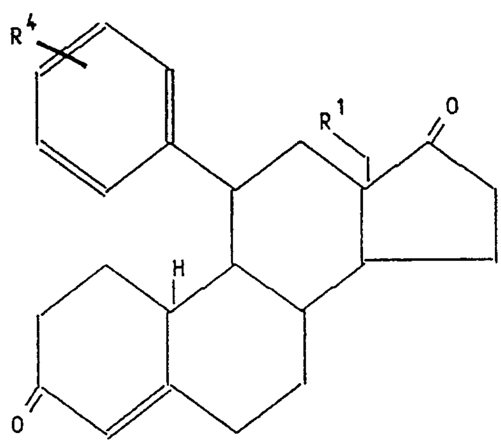

(Ia)

oder der Formel Ib

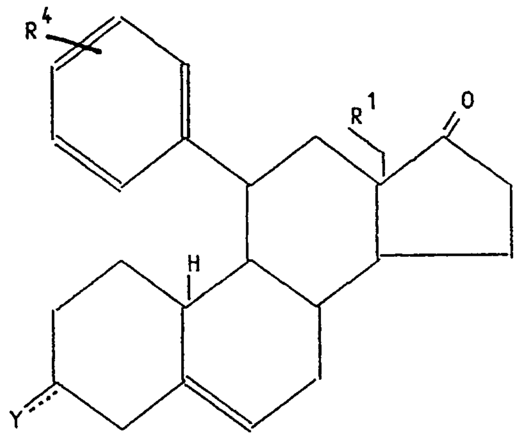

(Ib)

worin

$R^4$ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten $C_1$-$C_8$-Alkyl-, gegebenenfalls geschützten Acyl-oder Alkoxyalkylrest, für eine Aminogruppe

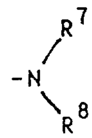

,

in welcher $R^7$ und $R^8$ unabhängig voneinander ein Wasserstoffatom oder eine $C_1$-$C_4$-Alkylgruppe bedeuten, oder für ein entsprechendes Aminoxid

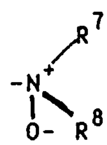

,

oder für die Gruppierungen $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα

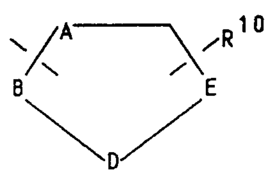

(Iα),

in welchem A ein Stickstoff-, Sauerstoff- oder Schwefelatom, -B-D-E- die Elementenfolge -C-C-C-, -N-C-C- oder -C-N-C- und $R^{10}$ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten $C_1$-$C_8$-Alkyl-, gegebenenfalls geschützten Acyl-oder Alkoxyalkylrest, für eine Aminogruppe

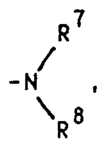

in welcher $R^7$ und $R^8$ unabhängig voneinander ein Wasserstoffatom oder eine $C_1$-$C_4$-Alkylgruppe bedeuten, oder ein entsprechendes Aminoxid

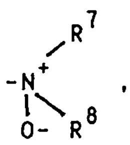

oder die Gruppierung $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten,
symbolisieren,
oder für einen Heteroarylrest der Formel I$\beta$

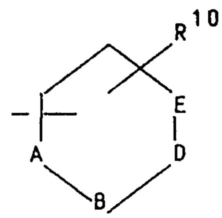

(Iβ),

in welchem A ein Stickstoffatom und -B-D-E- die Elementenfolge -C-C-C-, -N-C-C-, -C-N-C- oder -C-C-N- bedeuten und $R^{10}$ die bereits angegebene Bedeutung hat,
oder für einen Phenylrest der Formel I$\gamma$

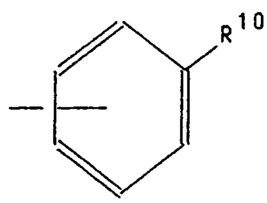

(Iγ),

worin $R^{10}$ die bereits angegebene Bedeutung hat,
stehen,
Y eine geschützte Ketogruppe oder eine geschützte Hydroxygruppe und ein Wasserstoffatom sowie
$R^1$ ein Wasserstoffatom oder eine Methylgruppe bedeuten,
dadurch gekennzeichnet, daß eine Verbindung der allgemeinen Formel III

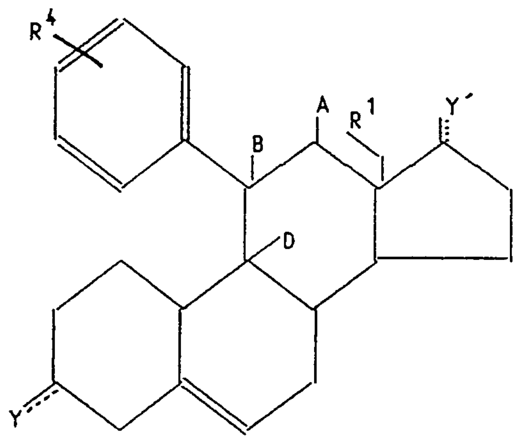

(III)

worin
A und B gemeinsam eine zusätzliche Bindung und
D ein Wasserstoffatom oder
B und D gemeinsam eine zusätzliche Bindung und
A ein Wasserstoffatom
bedeuten und
$R^4$, Y = Y' sowie $R^1$ die in den Formeln Ia bzw. Ib angegebene Bedeutung haben, zu einer Verbindung der allgemeinen Formel IIa

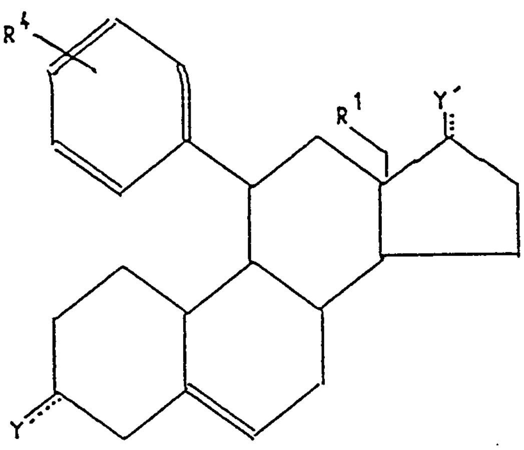

(IIa),

worin $R^4$, Y, Y' und $R^1$ die bereits angegebene Bedeutung haben, mit einem elektropositiven Metall in einem elektronensolvatisierenden Lösungsmittel reduziert und anschließend gegebenenfalls diese Verbindung der allgemeinen Formel IIa entweder mit Salzsäure, Schwefelsäure, Phosphorsäure, Toluolsulfonsäure oder einer anderen starken Mineralsäure zu einer Verbindung der allgemeinen Formel Ia oder mit Essigsäure, Oxalsäure oder einer anderen organischen Säure partiell zu einer Verbindung der allgemeinen Formel Ib gespalten wird.

2. Verfahren nach Anspruch 1, dadurch gekennzeichnet, daß mit Lithium, Natrium, Kalium, Calcium als elektropositivem Metall reduziert wird.

3. Verfahren nach Anspruch 2, dadurch gekennzeichnet, daß mit Lithium in flüssigem Ammoniak reduziert wird.

4. Verfahren nach Anspruch 1, dadurch gekennzeichnet, daß mit einem Überschuß des Metalls reduziert wird.

**5.** Verbindungen der allgemeinen Formel Ib

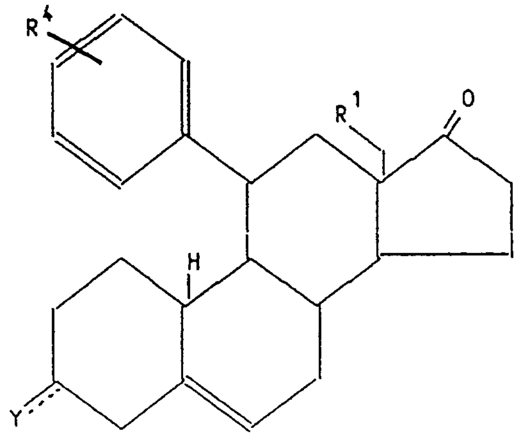

(Ib)

und Verbindungen der allgemeinen Formel IIa

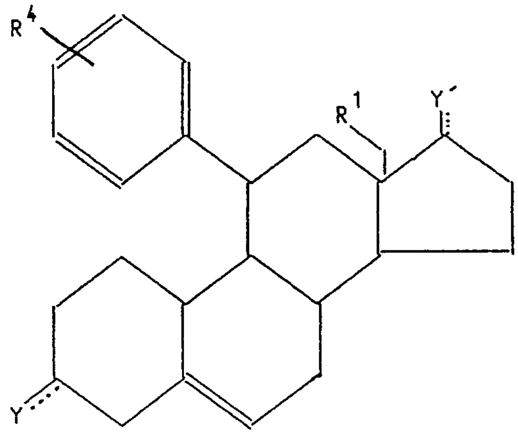

(IIa),

worin

$R^4$ für ein Wasserstoffatom, eine Cyanogruppe, ein Chlor-, Fluor-, Brom-. Jodatom, für eine Trialkylsilyl-, Trialkylstannylgruppe, für einen geradkettigen oder verzweigten, gesättigten oder ungesattigten $C_1$-$C_8$-Alkyl- gegebenenfalls geschützten Acyl-oder Alkoxyalkylrest, für eine Aminogruppe

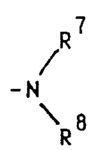

,

in welcher $R^7$ und $R^8$ unabhängig voneinander ein Wasserstoffatom oder eine $C_1$-$C_4$-Alkylgruppe bedeuten, oder für ein entsprechendes Aminoxid

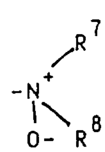

,

oder für die Gruppierungen $-OR^9$ oder $-S(O)_iR^9$ mit i = 0, 1 oder 2, in welchen $R^9$ ein Wasserstoff-

atom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα

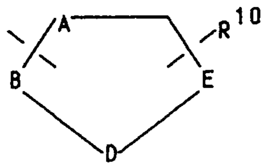

(Iα),

in welchem A ein Stickstoff-, Sauerstoff- oder Schwefelatom, -B-D-E- die Elementenfolge -C-C-C-, -N-C-C- oder -C-N-C- und $R^{10}$ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten $C_1$-$C_8$-Alkyl-, gegebenenfalls geschützten Acyl-oder Alkoxyalkylrest, für eine Aminogruppe

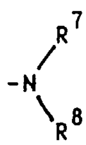

,

in welcher $R^7$ und $R^8$ unabhängig voneinander ein Wasserstoffatom oder eine $C_1$-$C_4$-Alkylgruppe bedeuten, oder ein entsprechendes Aminoxid

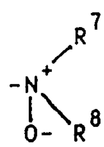

,

oder die Gruppierung -$OR^9$ oder -$S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten,
symbolisieren,
oder für einen Heteroarylrest der Formel Iβ

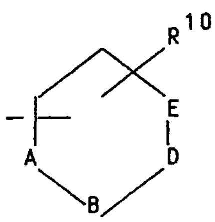

(IB),

in welchem A ein Stickstoffatom und -B-D-E- die Elementenfolge -C-C-C-, -N-C-C-, -C-N-C- oder -C-C-N- bedeuten und $R^{10}$ die bereits angegebene Bedeutung hat,
oder für einen Phenylrest der Formel Iγ

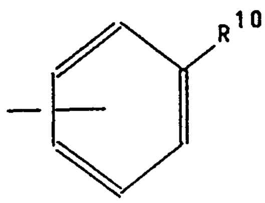

(Iγ),

worin $R^{10}$ die bereits angegebene Bedeutung hat,
stehen,

Y und Y' eine geschützte Ketogruppe oder eine geschützte Hydroxygruppe und ein Wasserstoffatom sowie

$R^1$ ein Wasserstoffatom oder eine Methylgruppe bedeuten.

## Claims

**1.** Process for the preparation of 10$\beta$-H-11$\beta$-(subst. phenyl)-steroids of formula Ia

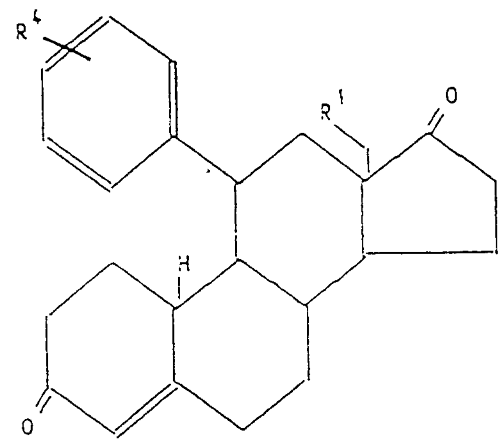

(Ia)

or formula Ib

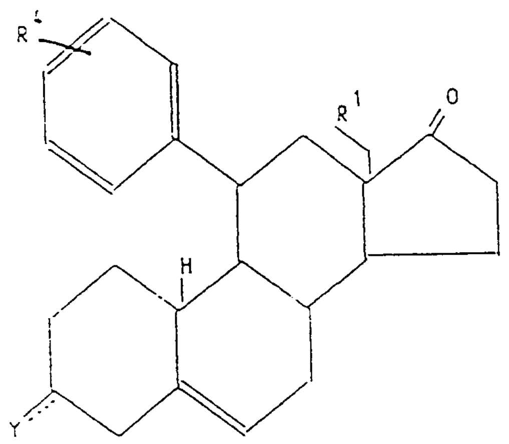

(Ib)

wherein
$R^4$ represents a hydrogen atom, a cyano group, a chlorine atom, a fluorine atom, a bromine atom, an iodine atom, a trialkylsilyl group, a trialkylstannyl group, a straight-chained or branched, saturated or unsaturated $C_1$-$C_8$-alkyl radical, an optionally protected acyl radical, an alkoxyalkyl radical, an amino group

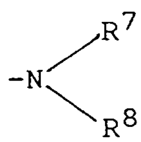

in which $R^7$ and $R^8$ each independently of the other represents a hydrogen atom or a $C_1$-$C_4$-alkyl group, or $R^4$ represents a corresponding amine oxide

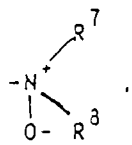

or the group $-OR^9$ or $-S(O)_iR^9$ in which i = 0, 1 or 2 and in which $R^9$ represents a hydrogen atom, a methyl group, an ethyl group, a propyl group, an isopropyl group, a methoxyphenyl group, an allyl group or a 2-dimethylaminoethyl group, or $R^4$ represents a heteroaryl radical of formula Iα

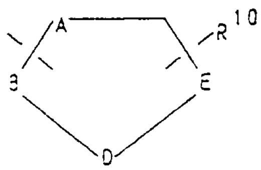

(Iα)

in which A represents a nitrogen atom, an oxygen atom or a sulphur atom, -B-D-E- represents the sequence of elements -C-C-C-, -N-C-C- or -C-N-C-, and $R^{10}$ representsa hydrogen atom, a cyano group, a chlorine atom, a fluorine atom, a bromine atom, an iodine atom, a trialkylsilyl group, a trialkylstannyl group, a straight-chained or branched, saturated or unsaturated $C_1$-$C_8$-alkyl radical, an optionally protected acyl radical, an alkoxyalkyl radical, an amino group

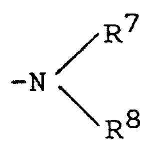

in which $R^7$ and $R^8$ each independently of the other represents a hydrogen atom or a $C_1$-$C_4$-alkyl group, or $R^{10}$ represents a corresponding amine oxide

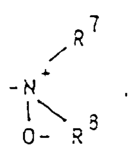

or the group $-OR^9$ or $-S(O)_iR^9$ in which i = 0, 1 or 2 and in which $R^9$ represents a hydrogen atom, a methyl group, an ethyl group, a propyl group, an isopropyl group, a methoxyphenyl group, an ally] group or a 2-dimethylaminoethyl group,
or $R^4$ represents a heteroaryl radical of formula Iβ

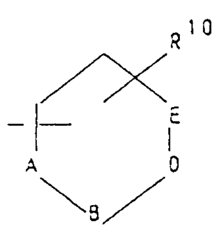

(Iß)

in which A represents a nitrogen atom and -B-D-E-represents the sequence of elements -C-C-C-, -N-C-C-, -C-N-C-or -C-C-N-, and $R^{10}$ has the meaning given above, or $R^4$ represents a phenyl radical of formula Iγ

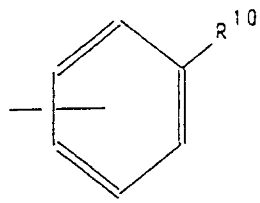

($I_\gamma$)

in which $R^{10}$ has the meaning given above;
Y represents a protected keto group or a protected hydroxy group and a hydrogen atom; and
$R^1$ represents a hydrogen atom or a methyl group,
characterised in that a compound of the general formula III

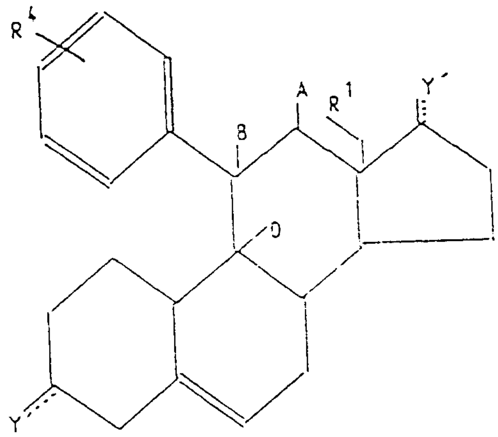

(III),

wherein
A and B together represent an additional bond and
D represents a hydrogen atom, or
B and D together represent an additional bond and
A represents a hydrogen atom, and
$R^4$, Y = Y' and $R^1$ have the meaning given in formulae Ia and Ib,
is reduced by means of an electropositive metal in an electron-solvating solvent to a compound of the general formula IIa

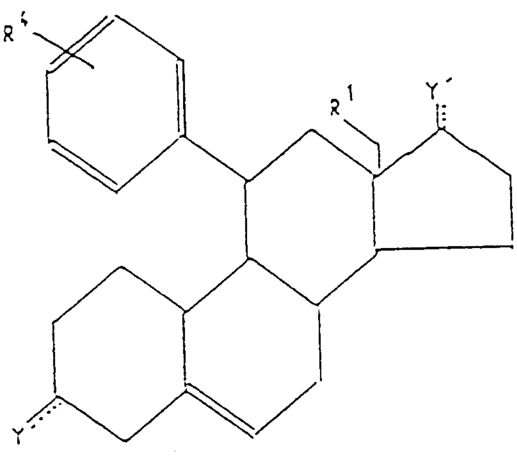

(IIa)

wherein $R^4$, Y, Y' and $R^1$ have the meaning given above, and then, optionally, the compound of the general formula IIa is either cleaved with hydrochloric acid, sulphuric acid, phosphoric acid, toluenesulphonic acid or another strong mineral acid to form a compound of the general formula Ia, or is partially

cleaved with acetic acid, oxalic acid or another organic acid to form a compound of the general formula Ib.

**2.** Process according to claim 1, characterised in that the reduction is carried out with lithium, sodium, potassium or calcium as the electropositive metal.

**3.** Process according to claim 2, characterised in that the reduction is carried out with lithium in liquid ammonia.

**4.** Process according to claim 1, characterised in that the reduction is carried out with an excess of the metal.

**5.** Compounds of the general formula Ib

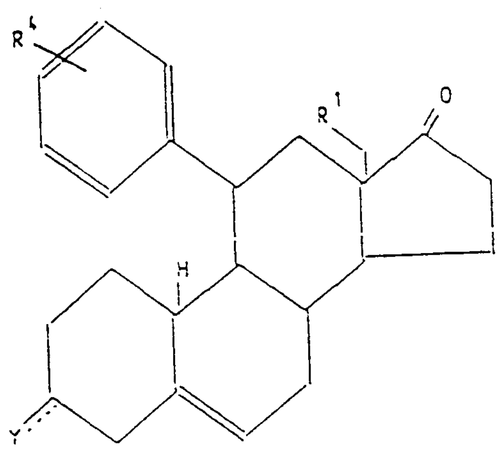

(Ib)

and compounds of the general formula IIa

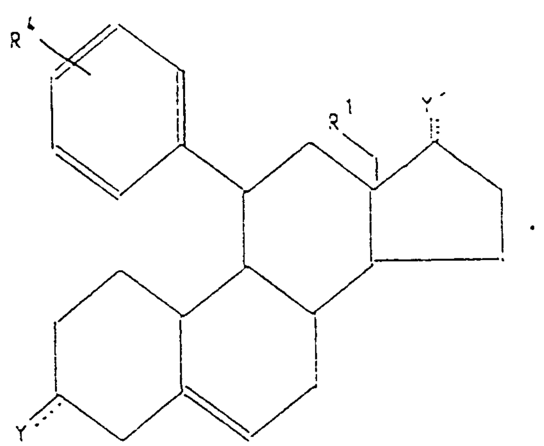

(IIa)

wherein

$R^4$ represents a hydrogen atom, a cyano group, a chlorine atom, a fluorine atom, a bromine atom, an iodine atom, a trialkylsilyl group, a trialkylstannyl group, a straight-chained or branched, saturated or unsaturated $C_1$-$C_8$-alkyl radical, an optionally protected acyl radical, an alkoxyalkyl radical, an amino group

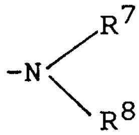

in which $R^7$ and $R^8$ each independently of the other represents a hydrogen atom or a $C_1$-$C_4$-alkyl group, or $R^4$ represents a corresponding amine oxide

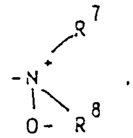

or the group $-OR^9$ or $-S(O)_iR^9$ in which i = 0, 1 or 2 and in which $R^9$ represents a hydrogen atom, a methyl group, an ethyl group, a propyl group, an isopropyl group, a methoxyphenyl group, an allyl group or a 2-dimethylaminoethyl group, or $R^4$ represents a heteroaryl radical of formula Iα

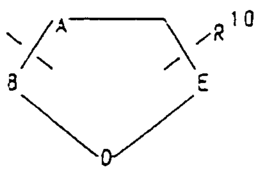

(Iα)

in which A represents a nitrogen atom, an oxygen atom or a sulphur atom, -B-D-E- represents the sequence of elements -C-C-C-, -N-C-C- or -C-N-C-, and $R^{10}$ representsa hydrogen atom, a cyano group, a chlorine atom, a fluorine atom, a bromine atom, an iodine atom, a trialkylsilyl group, a trialkylstannyl group, a straight-chained or branched, saturated or unsaturated $C_1$-$C_8$-alkyl radical, an optionally protected acyl radical, an alkoxyalkyl radical, an amino group

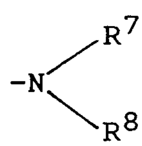

in which $R^7$ and $R^8$ each independently of the other represents a hydrogen atom or a $C_1$-$C_4$-alkyl group, or $R^{10}$ represents a corresponding amine oxide

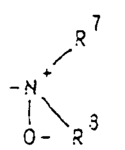

or the group $-OR^9$ or $-S(O)_iR^9$ in which i = 0, 1 or 2 and in which $R^9$ represents a hydrogen atom, a methyl group, an ethyl group, a propyl group, an isopropyl group, a methoxyphenyl group, an ally] group or a 2-dimethylaminoethyl group,
or $R^4$ represents a heteroaryl radical of formula Iβ

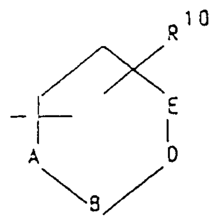

(Iß)

in which A represents a nitrogen atom and -B-D-E-represents the sequence of elements -C-C-C-, -N-C-C-, -C-N-C- or -C-C-N-, and $R^{10}$ has the meaning given above, or $R^4$ represents a phenyl radical of formula Iγ

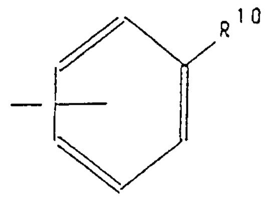

(Iγ)

in which $R^{10}$ has the meaning given above;
Y and Y' represent a protected keto group or a protected hydroxy group and a hydrogen atom; and
$R^1$ represents a hydrogen atom or a methyl group.

## Revendications

1. Procédé de préparation de 10β-H-11β-phénylstéroïdes de formule Ia

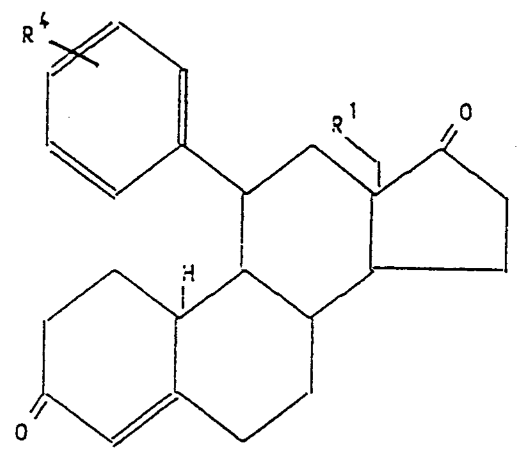

(Ia)

ou de formule Ib

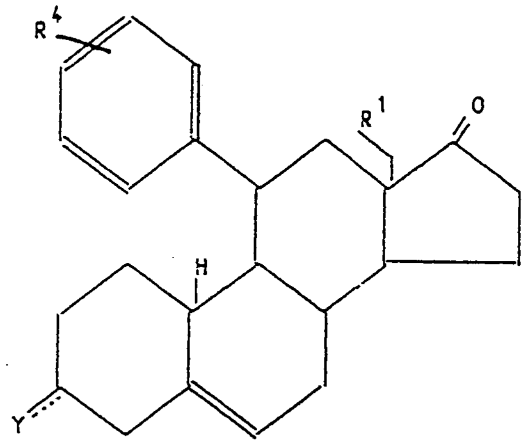

(Ib)

formules dans lesquelles
$R^4$ représente un atome d'hydrogène, un groupe cyano, un atome de chlore, de fluor, de brome ou d'iode, un groupe trialkylsilyle ou trialkylstannyle, un alkyle en $C_1$-$C_8$ linéaire ou ramifié, saturé ou insaturé, un acyle éventuellement protégé ou un alcoxyalkyle, un groupe amino

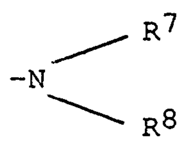

dont $R^7$ et $R^8$ sont chacun, indépendamment l'un de l'autre, un atome d'hydrogène ou un alkyle en $C_1$-$C_4$, ou bien un aminoxyde

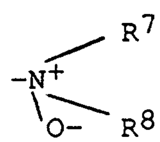

correspondant,
ou un groupe $-OR^9$ ou $-S(O)_iR^9$ avec i = 0, 1 ou 2, $R^9$ désignant un atome d'hydrogène ou un groupe méthyle, éthyle, propyle, isopropyle, méthoxyphényle, allyle ou 2-diméthylaminoéthyle, ou encore un hétéroaryle de formule Iα

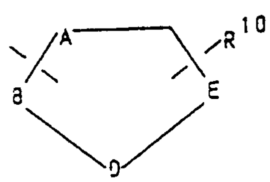

(Iα),

A représentant un atome d'azote, d'oxygène ou de soufre, -B-D-E- la suite -C-C-C-, -N-C-C- ou -C-N-C- et $R^{10}$ un atome d'hydrogène, un groupe cyano, un atome de chlore, de fluor, de brome ou d'iode, un groupe trialkylsilyle ou trialkylstannyle, un alkyle en $C_1$-$C_8$ linéaire ou ramifié, saturé ou insaturé, un acyle éventuellement protégé ou un alcoxyalkyle,
un groupe amino

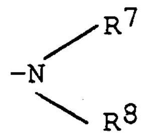

dont $R^7$ et $R^8$ sont chacun,
indépendamment l'un de l'autre, un atome d'hydrogène ou un alkyle en $C_1$-$C_4$, ou un aminoxyde correspondant

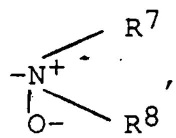

ou encore un groupe -$OR^9$ ou -$S(O)_iR^9$ avec i = 0, 1 ou 2, $R^9$ désignant un atome d'hydrogène ou un groupe méthyle, éthyle, propyle, isopropyle, méthoxyphényle, allyle ou 2-diméthylaminoéthyle,
ou encore un hétéroaryle de formule I$\beta$

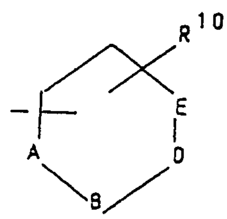

(Iß),

A représentant un atome d'azote et -B-D-E- la suite -C-C-C-, -N-C-C-, -C-N-C- ou -C-C-N-, et $R^{10}$ ayant la signification précédemment indiquée,
ou un radical phényle de formule I$\gamma$

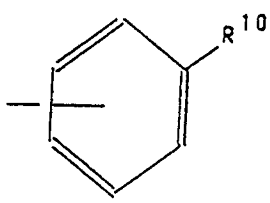

(I$\gamma$),

$R^{10}$ ayant également la signification précédemment indiquée,
Y représente un groupe céto protégé ou un groupe hydroxy protégé et un atome d'hydrogène et
$R^1$ un atome d'hydrogène ou le groupe méthyle,
procédé caractérisé en ce que l'on réduit un composé de formule générale III

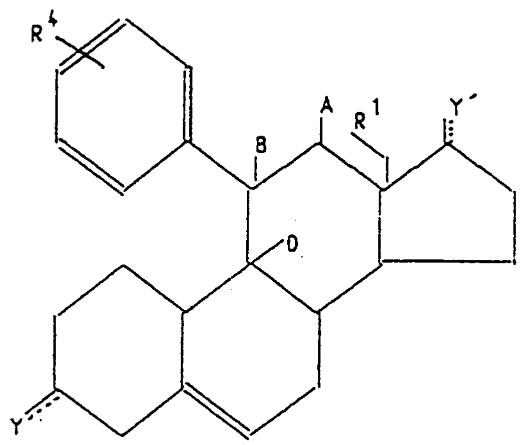

(III)

(dans laquelle A et B représentent ensemble une liaison supplémentaire et
D un atome d'hydrogène ou bien
B et D une liaison supplémentaire et
A un atome d'hydrogène,
les autres symboles ayant les mêmes significations que dans les formules Ia et Ib et Y = Y')
en un composé de formule générale IIa

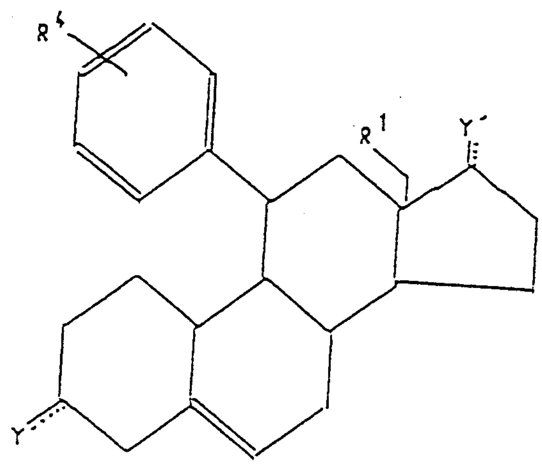

(IIa),

les divers symboles ont les significations précédentes, en opérant avec un métal électropositif dans un solvant de solvatation électronique, puis le cas échéant on scinde ce composé IIa en un composé de formule Ia avec de l'acide chlorhydrique, sulfurique, phosphorique, toluène-sulfonique ou un autre acide minéral fort, ou bien on le scinde partiellement en un composé de formule Ib avec de l'acide acétique ou oxalique ou un autre acide organique.

**2.** Procédé selon la revendication 1, caractérisé en ce que l'on effectue la réduction avec du lithium, du sodium, du potassium ou du calcium comme métal électropositif.

**3.** Procédé selon la revendication 2, caractérisé en ce que l'on effectue la réduction avec du lithium dans de l'ammoniac liquide.

**4.** Procédé selon la revendication 1, caractérisé en ce que l'on opère avec un excès du métal.

**5.** Les composés de formule générale Ib

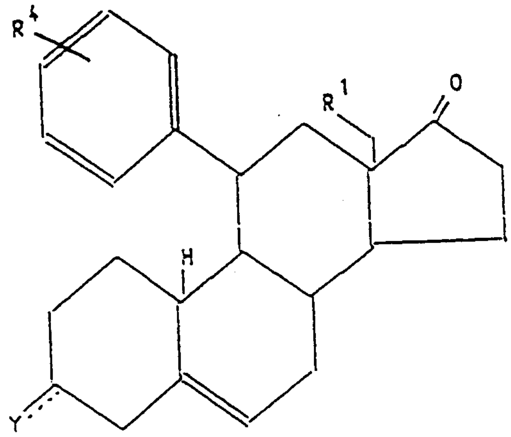

(Ib)

et ceux de formule générale IIa

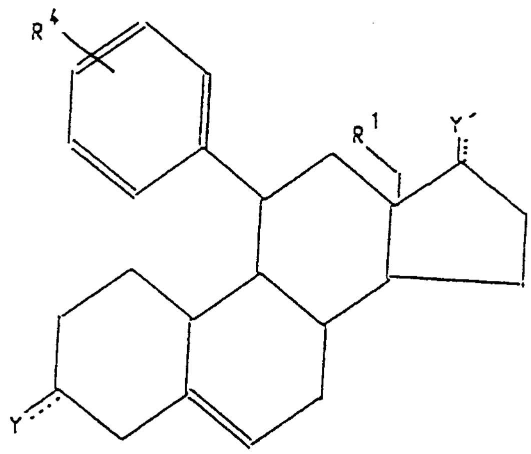

(IIa),

formules dans lesquelles

$R^4$ représente un atome d'hydrogène, un groupe cyano, un atome de chlore, de fluor, de brome ou d'iode, un groupe trialkylsilyle ou trialkylstannyle, un alkyle en $C_1$-$C_8$ linéaire ou ramifié, saturé ou insaturé, un acyle éventuellement protégé ou un alcoxyalkyle, un groupe amino

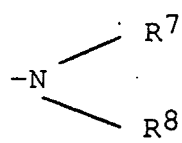

dont $R^7$ et $R^8$ sont chacun, indépendamment l'un de l'autre, un atome d'hydrogène ou un alkyle en $C_1$-$C_4$, ou bien un aminoxyde

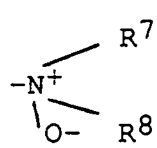

correspondant,

ou un groupe $-OR^9$ ou $-S(O)_iR^9$ avec i, = 0, 1 ou 2, $R^9$ désignant un atome d'hydrogène ou un groupe méthyle, éthyle, propyle, isopropyle, méthoxyphényle, allyle ou 2-diméthylaminoéthyle, ou encore un hétéroaryle de formule Iα

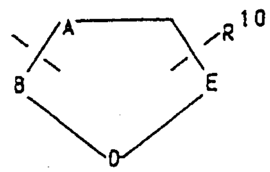

( Iα ),

A représentant un atome d'azote, d'oxygène ou de soufre, -B-D-E- la suite -C-C-C-, -N-C-C- ou -C-N-C- et $R^{10}$ un atome d'hydrogène, un groupe cyano, un atome de chlore, de fluor, de brome ou d'iode, un groupe trialkylsilyle ou trialkylstannyle, un alkyle en $C_1$-$C_8$ linéaire ou ramifié, saturé ou insaturé, un acyle éventuellement protégé ou un alcoxyalkyle,
un groupe amino

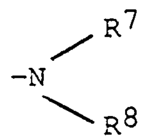

dont $R^7$ et $R^8$ sont chacun,
indépendamment l'un de l'autre, un atome d'hydrogène ou un alkyle en $C_1$-$C_4$, ou un aminoxyde correspondant

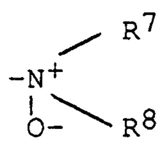

,

ou encore un groupe $-OR^9$ ou $-S(O)_iR^9$ avec i = 0, 1 ou 2, $R^9$ désignant un atome d'hydrogène ou un groupe méthyle, éthyle, propyle, isopropyle, méthoxyphényle, allyle ou 2-diméthylaminoéthyle,
ou encore un hétéroaryle de formule Iβ

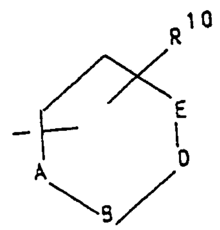

( Iβ ),

A représentant un atome d'azote et -B-D-E- la suite -C-C-C-, -N-C-C-, -C-N-C- ou -C-C-N-, et $R^{10}$ ayant la signification précédemment indiquée,
ou un radical phényle de formule Iγ

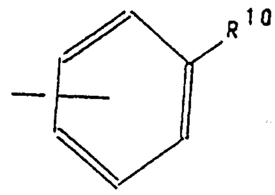

( Iγ ),

$R^{10}$ ayant également la signification précédemment indiquée,
Y représente un groupe céto protégé ou un groupe hydroxy protégé et un atome d'hydrogène et

$R^1$ un atome d'hydrogène ou le groupe méthyle.